# EUROPEAN PATENT SPECIFICATION

(11) **EP 2 219 574 B1**
(45) Date of publication and mention of the grant of the patent: **29.01.2014**
(21) Application number: 07852164.8
(22) Date of filing: 22.11.2007
(51) Int. Cl.: A61F 13/02, B32B 7/06

(54) **FILM DRESSING WITH AN IMPROVED GRIP TAB**
FOLIENVERBAND MIT VERBESSERTER GREIFLASCHE
PANSEMENT EN FILM AVEC UNE LANGUETTE DE SAISIE AMÉLIORÉE

(43) Date of publication of application: 25.08.2010
(73) Proprietor: Mölnlycke Health Care AB, 402 52 Göteborg (SE)
(72) Inventor: FABO, Tomas, S-435 39 Mölnlycke (SE); SVENSBY, Anna, S-426 77 Västra Frölunda (SE); HANSSON, Dennis, S-424 56 Gunnilse (SE)
(74) Representative: Börlin, Maria
(86) International application number: PCT/SE2007/050892
(87) International publication number: WO 2009/067062

(56) References cited:
- EP-A1- 0 507 459
- EP-A2- 0 066 899
- EP-A2- 0 144 891
- EP-A2- 0 750 892
- US-A- 4 619 253
- US-A- 5 106 629
- US-A- 5 520 629
- US-A- 5 599 289

## Description

### Technical field

The present invention relates to a film dressing comprising a plastic film, which is coated on one side with a skin-friendly adhesive, a backing layer, which is detachably affixed to the plastic film on the opposite side to the adhesive-coated side, and a protective layer detachably affixed to the adhesive coating.

### Background of the invention

At the beginning of the 1980s Smith & Nephew, a company which manufactures wound care products, launched a new type of wound dressing. The product, which continues to be marketed and sold under the name OpSite™, is based on an invention described in the British patent GB 1280631. The dressing consists of a very thin polyurethane film, approximately 25 micrometres thick, which is coated with a thin self-adhesive layer, also approximately 25 micrometres thick. Its thinness makes the film very flexible and pliable. It can be affixed and closed relatively tightly around wounds on uneven skin surfaces, both convex and concave. Any wrinkles that are formed, however, sometimes produce narrow channels through which fluid can leak. This dressing, which was quite unlike all previous dressing products, formed an altogether new category of self-adhesive film dressings. Several companies followed suit with similar products, and nowadays there is a large number of film products with a similar function and similar characteristics on the market. These film dressings soon found an important application in the fixing and protection of i.v. cannulas (of the Venflon cannula type, for example). They are also used for covering various types of wounds, such as the sites from which skin is taken for skin transplants, for example.

Besides the aforementioned characteristics, an important prerequisite for the great market success of the film products was the successful development of good application systems. The thin dressings are very difficult to handle without specially designed application systems. A stiffer carrier material is therefore normally detachably affixed to the non-adhesive side of the film in order to stabilize the product, which would otherwise easily become tangled up when applying it to the skin. The carrier material that stabilizes the thin film is separated from the product only once the self-adhesive film has been applied to the intended site. On the adhesive-coated side of the product is another protective film, a so-called release film, which is intended to protect the adhesive. Before the product is applied to the body, the release material is pulled off the product. After applying the product to the body, the backing carrier material is pulled off the non-adhesive-coated side.

The original solution was gradually improved in that the stiff carrier material was made to extend only over peripheral parts of the product like a frame, instead of covering the entire surface of the film. This rendered the product more deformable before the carrier material had been removed and made it easier to place this over convex or other uneven parts of the body. In order to facilitate removal of the frame once the product was affixed to the skin, the frame but not the film was provided with one or more punched sections or break lines. These break lines made it easier to grasp the frame with the fingernails or finger tips, making it easier to remove the frame.

Later on, the film dressing with backing frame was further developed in various ways. Specially designed i.v. affixing films have been developed with a punched, narrow slit to allow space for the tube connection of the i.v. cannula, so as to improve the functioning. A part of the film dressing has been provided with a wound pad, which covers the centre part of the adhesive-coated surface (so-called island dressing), so as to afford a certain absorption of wound fluid when the film dressing is applied over the wound. Film dressings are also used as integral components of a stoma dressing in proximity to the skin.

EP 0507459 relates to a combined adhesive strip and transparent dressing. EP 0507459 discloses a dressing including a backing layer coated with a pressure-sensitive adhesive, a liner which cover the adhesive, and a carrier window and carrier frame attached to the backing layer to provide support and rigidity to the backing layer. The carrier frame includes a grip tab which protrudes from an edge of the dressing, wherein a break line is provided in the carrier frame at a position where the carrier frame does not protrude outside the adhesive coating.

EP0750892 relates to a dressing comprising an absorbent adhesive barrier layer, a backing layer and a release sheet covering a skin-contacting surface of the barrier layer. The release sheet includes a predetermined line of separation dividing the release sheet into two separable sections, each of said section having a grip tab, which extends beyond a perimeter of the skin-contacting surface.

US 4,619,253 relates to a foil- or film-like bandage consisting of a transparent continuous foil which on the one side is covered with a transparent supporting foil and on the other side is provided with an adhesive layer and a removable cover layer thereupon. The cover layer having the same size as the adhesive layer and having preset cuts or breaking lines between two points at different edges of the cover layer.

Known film dressings have proved to have several deficiencies, however.

It can be very difficult to grasp the frame at the break lines with the fingernails or finger tips when the frame is to be removed. Attempts to do so often mean that the film is partially raised, which can lead to the film becoming wrinkled and not adhering as well. There is also a risk of the film being damaged when inserting the fingernails under the frame at the break lines so as to lift this from the film.

The object of the present invention is to eliminate these problems and to provide a film dressing that is easier to apply than currently known film dressings.

### Summary of the invention

This object is achieved by means of a film dressing comprising a plastic film, which is coated on one side with a skin-friendly adhesive, a backing layer, which is detachably affixed to the plastic film on the opposite side to the adhesive-coated side, and a protective layer detachably affixed to the adhesive coating, characterized in that the backing layer comprises at least one grip tab, which protrudes from an edge of the dressing beyond the outline of the plastic film, and that a break line extends from the outer edge of each grip tab towards a central part of the backing layer, dividing the grip tab into two parts. The fact that the backing layer is not joined to the film in said area makes it easy to grasp the backing layer without any risk that the applied film will become detached from the underlying surface and possibly creased. At the same time, the absence of film under this area helps to prevent accidental interference with the film when grasping the backing layer in this area. The arrangement of break lines means that there is no need to remove the entire frame in one piece, parts of the frame instead being removed separately, which facilitates the application of the dressing.

In a preferred embodiment the backing layer has a central opening. Such a frame makes the dressing easier to apply to a patient than one fully covered by a backing layer. The protective layer preferably has an outline corresponding to the outline of the backing layer. An easily distinguishable grip tab is thereby also formed in the protective layer.

In one variant the break line in the portion that passes through a grip tab, which extends beyond the outline of the plastic film, consists of a discontinuous break line.

In a preferred embodiment a grip tab protrudes from two opposite edges of the dressing and a break line extends from the outer edge of each grip tab to the central opening in the backing layer on at least two opposite sides thereof, these break lines being arranged so that an imaginary extension of one of these break lines does not coincide with the other break line.

In one variant a curved or wavy break line extends from the outer edge of each grip tab to the central opening in the backing layer. This serves to prevent the break lines forming fold marks on the frame, which can cause the dressing to be inadvertently folded during handling, which would make the application more difficult.

### Brief description of drawings

The invention will now be described with reference to the drawings attached, in which:
Fig. 1 shows a schematic plan view from above of a film dressing according to a first preferred embodiment of the invention,
Fig. 2 shows a cross sectional view along the line II-II in Fig. 1,
Fig. 3 shows a schematic plan view from above of a part of a dressing according to a second preferred embodiment of the invention,
Fig. 4 shows a schematic plan view from above of a film dressing according to a third embodiment of the invention,
Fig. 5 shows a schematic plan view from above of a film dressing according to a fourth embodiment of the invention,
Fig. 6 schematically represents a stoma bandage, of which a film dressing according to the invention forms a constituent component, in a cross sectional view along the line VI-VI in Fig. 7, and
Fig. 7 shows a plan view of the stoma bandage in Fig. 6 with the stoma bag removed.

### Description of embodiments

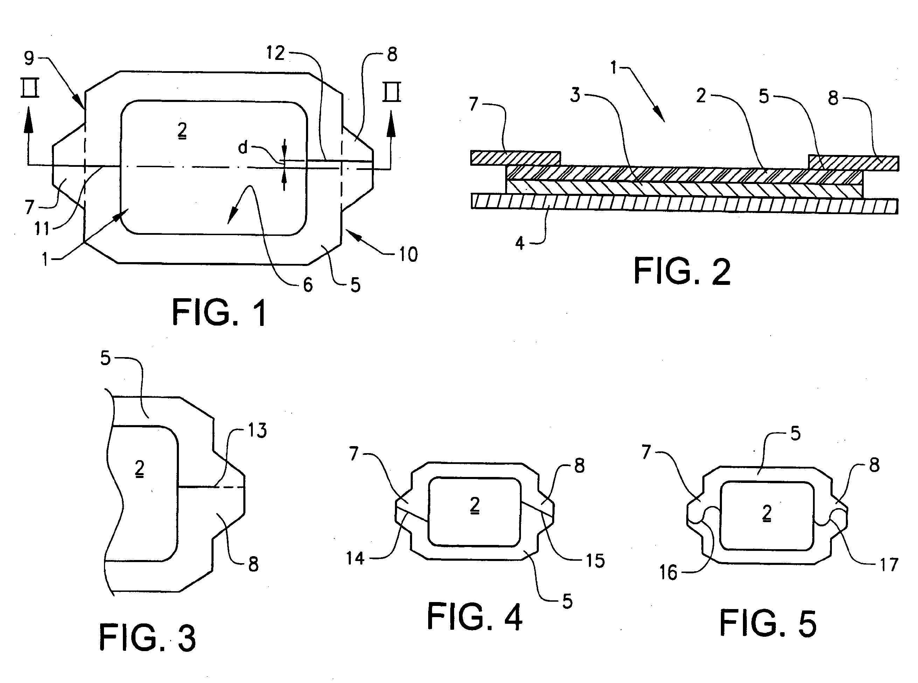

Figs. 1 and 2 show a film dressing 1 according to a first preferred embodiment of the invention. The dressing 1 conventionally comprises a plastic film 2, which on its underside is coated with an adhesive layer 3, a protective layer 4 on the underside of the adhesive coating 3 and a backing layer 5 on the upper side of the plastic film 2.

The terms "upper side" and "underside" and similar designations in the description relate to the reciprocal relationships between the components of the dressing in the position of the dressing shown in Fig. 1.

The protective layer 4 is detachably affixed to the adhesive coating 3. The function of the protective layer 4 is to protect the adhesive coating prior to application, so that particles or other impurities do not adhere to the adhesive coating 3, thereby impairing the adhesion. The protective layer 4 furthermore prevents the dressing from accidentally adhering to the incorrect place when handling the dressing prior to the intended application on a patient. Since the protective layer must be removed when applying the dressing, this attachment to the dressing must be a loose one.

The backing layer 5 is detachably affixed to the upper side of the plastic film 2. The function of the backing layer 5 is to facilitate handling of the dressing prior to application and during the application thereof. Without the backing layer, the thin, adhesive-coated plastic film is in practice virtually impossible to handle and to apply without the film folding or wrinkling. Since the backing layer must be removed after applying the dressing, this adhesion to the film 2 must be weaker than the adhesion of the adhesive layer 3 of the plastic film to the skin after application on a patient. In addition the internal cohesion of the adhesive layer must be greater than the adhesion of the backing layer 5 to the upper side of the plastic film, in order that the plastic film will not come away with the backing layer when this is removed, leaving a part of the adhesive layer on the skin. The adhesion of the backing layer 5 to the upper side of the plastic film must nevertheless be greater than the adhesion of the protective layer 4 to the adhesive layer 3, in order that the protective layer can be removed without the risk of the backing layer 5 becoming detached instead of the protective layer.

In the preferred embodiment the backing layer 5 has a central opening 6, so that the backing layer 5 forms a frame, which extends around the outer parts of the plastic film 2. The dressing 1 furthermore has two grip tabs 7, 8, which protrude from two opposite edges 9, 10 of the dressing, from the left-hand and right-hand edge respectively in Figs. 1 and 2. These grip tabs comprise the areas extending beyond the outline of the adhesive-coated plastic film 2, as can be seen from Figs. 1 and 2. The absence of the plastic film under the grip tabs means that after application of the dressing these can easily be grasped without a risk of the plastic film wrinkling or becoming detached from the underlying surface. In know film dressings, in which the backing layer is removed by detaching the backing layer at the break lines, it is difficult to grasp the backing layer by inserting the fingernails or finger tips under the backing layer at these break lines. In the case of the known dressings there is a great risk of the applied film being locally detached from the underlying surface or of fingernails damaging the film. Forming grip tabs which extend beyond the outline of the plastic film, that is to say outside the periphery thereof, eliminates these risks. In addition, the absence of film under the tabs makes it much easier to grasp the backing layer 5 than has hitherto been the case.

As can be seen from Fig. 2, the protective layer 4 also has areas that extend beyond the outline of the adhesive-coated plastic film 2. In the embodiment shown in Figs 1 and 2, the protective layer 4 has a similar outline to the backing layer 5 and hence grip tabs corresponding to the grip tabs 7, 8.

The backing layer 5 is divided into two parts by break lines 11, 12, which extend from the outer edge of each grip tab 7, 8 to the central opening 6. The break lines 11, 12 extend through the entire thickness of the backing layer 5 and divide this into two parts. As can be seen from Fig. 1, these break lines 11, 12 are laterally offset in relation to one another by a distance d, so that an imaginary extension of one of the lines 11, 12 does not coincide with the other line. Such a design serves to prevent the break lines forming a fold mark or folding axis, around which the dressing might inadvertently be folded when handling it during its application. Such folding might cause parts of the dressing to adhere to itself, which naturally makes it more difficult or impossible to apply the dressing. The distance d is preferably greater than 2 mm.

Figs. 3, 4 and 5 show further embodiments of the film dressing according to the invention. These embodiments differ from the embodiment according to Figs. 1 and 2 only in that the break lines are formed differently. Otherwise the constituent components of the dressing are identical and have therefore been given the same reference numerals as corresponding components in Figs. 1 and 2.

The break lines in the film dressing shown in Fig. 3, of which only the right-hand break line 13 is shown in Fig. 3, differ from the break lines occurring in the first embodiment shown in Figs. 1 and 2 only in that the break lines inside the area of the grip tabs are discontinuous. The break line 13 shown in Fig. 3 therefore has an outer portion, which constitutes a perforation line, that is to say the break line has a number of interruptions, in which the two parts of the backing layer defined by the break lines hang together. Such a design reduces the risk of inadvertently detaching either of the two parts of the backing layer.

In the embodiment shown in Fig. 4 the break lines 14, 15 on each grip tab run obliquely to the longitudinal and transverse direction of the dressing and are located so that imaginary extensions of the break lines do not coincide with one another. Otherwise the dressing in Fig. 4 is formed in the same way as the dressing in Figs. 1 and 2.

In the embodiment shown in Fig. 5 the break lines 16, 17 are wavy and can therefore not form fold marks. Otherwise the dressing in Fig. 5 is formed in the same way as the dressing in Figs. 1 and 2. It is naturally possible to give the break lines 16, 17 some other wavy form, for example in the shape of a square wave, or any desired curved shape. A curved shape inherently helps to prevent the occurrence of fold marks.

It is naturally possible to endow the break lines in the dressing in the embodiments according to Figs. 1, 4 and 5 with discontinuous portions like the break lines in Fig. 3.

The plastic film 2 preferably consists of a polyurethane film having a thickness of 10 to 50 micrometres. However, all the plastic films used in known film dressings can be used in a film dressing according to the invention.

Suitable soft, skin-friendly adhesives suitable for use in the adhesive layer 3 according to the invention may be composed, for example, of addition-curing RTV (room temperature vulcanizing) silicone systems, which after mixing cross-link to form a self-adhesive elastomer. Examples of RTV addition-curing silicone systems are specified in EP 0 300 620 A1, which describes so-called gel-forming compositions composed of an alkenyl-substituted polydiorganosiloxane, an organosiloxane containing hydrogen atoms linked to a proportion of the silicone atoms and a platinum catalyst.

Wacker SilGel 612 is a commercially available RTV silicone system. This is a two-component system. By varying the relative proportions of the two components A:B from 1.0:0.7 to 1.0:1.3, it is possible to vary the softness and the level of adhesion of the elastomer formed.

Examples of further soft, skin-friendly silicone elastomers are NuSil MED-6340, NuSil MED3-6300, NuSil MED12-6300 from NuSil Technology, Carpintieria, GA, USA and Dow Coming 7-9800 from Dow Coming Corporation, Midland, USA.

Other soft, skin-friendly adhesives are also feasible for the present invention, for example molten adhesive like Dispomelt ® 70-4647 from National Starch and Chemical Company, Bridgewater, NJ, USA.

However, all adhesives used in known film dressings can be used in a dressing according to the invention.

The protective layer 4, which is also often referred to as the release layer, depends on the choice of adhesive. For the silicone-based adhesives preferred according to the present invention, the protective layer 4 is suitably composed of polythene. For other types of adhesive the release layers that are used in known film dressings can also be used in dressings according to the present invention.

The material in the backing layer may advantageously be composed of polythene or a polythene and paper laminate. However, all materials used for the backing layer of known film dressings may be used in a dressing according to the present invention.

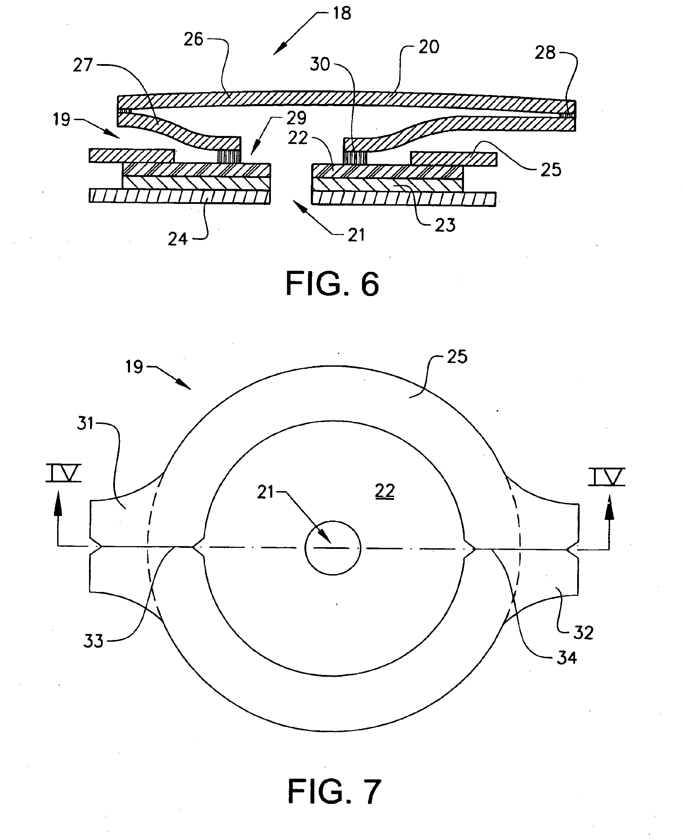

Fig. 6 in a cross sectional view shows a stoma bandage 18, which comprises a film dressing 19 and a stoma bag 20 affixed to the upper side of the film dressing. Fig. 7 shows the stoma bandage 18 with stoma bag removed in order to show the design of the film dressing 19 more clearly.

Apart from having a stoma bag 20 affixed thereto, the film dressing 19 differs from film dressings hitherto described only in that it has a circular rather than a rectangular shape and that it has a hole 21 at its centre. As in the other embodiments, the film dressing 19 includes a plastic film 22, which is coated on its underside with an adhesive layer 23, a protective layer 24, which is affixed so that it is easily detachable from the adhesive layer 23, and a backing layer 25, which must be removed when applying the stoma bandage. The same materials as can be used in film dressings previously described are also suitable for the film dressing 19.

The stoma bag 20 comprises two plastic films 26, 27, which are joined to one another around their periphery by a fused or adhesive joint 28. The lower film 27 contains a hole 29, which surrounds the hole 21 in the plastic film 22 of the film dressing. The edge around the hole 29 in the film 27 is affixed by means of a fused or adhesive joint 30 to the upper side of the plastic film 22 of the film dressing.

The backing layer 25 extends beyond the plastic film 22 coated with the adhesive layer 23 on two diametrically opposite sides. The parts of the backing layer that extend beyond the plastic film 22 of the film dressing 19 comprise grip tabs 31, 32. As in the embodiments previously described, each grip tab 31, 32 of the backing layer 25 is divided into two parts by a break line 33, 34, which extends inwards from the outer edge of the grip tab to the inner edge of the backing layer 25. In the embodiment shown, extensions of the break lines 33, 34 intersect, but they are preferably designed as in other embodiments shown so that their extensions do not intersect.

The embodiments described can obviously be modified without departing from the scope of the invention. For example, the dressing may have a shape other than the shape shown in the drawings, for example a circular or oval shape. The backing layer may be without an opening, that is to say extending over the entire surface of the plastic film, in which case it is preferably transparent, a break line preferably defining a central part of the backing layer. The dressing may also consist of an elongated dressing which is intended to be cut off in a length suited to the particular application and which has rows of corresponding grip tabs according to the invention along its longitudinal sides. It is also feasible to provide the dressing with just one single grip tab or more than two grip tabs. The grip tabs may also conceivably extend along the entire adjoining edge of the plastic film. More than two break lines can furthermore be arranged to define more than two separately removable parts of the backing layer. The invention shall therefore be limited solely by the content of the patent claims attached.

## Claims

1. Film dressing comprising a plastic film (2), which is coated on one side with a skin-friendly adhesive (3), a backing layer (5), which is detachably affixed to the plastic film on the opposite side to the adhesive-coated side, and a protective layer (4) detachably affixed to the adhesive coating, **characterized in that** the backing layer (5) comprises at least one grip tab (7, 8), which protrudes from an edge of the dressing beyond the outline of the plastic film, and that a break line (11, 12) extends from the outer edge of each grip tab (7, 8) towards a central part (6) of the backing layer, dividing the grip tab into two parts.

2. Film dressing according to Claim 1, **characterized in that** the backing layer (5) has a central opening (6).

3. Film dressing according to Claim 2, **characterized in that** the protective layer (4) has an outline corresponding to the outline of the backing layer (5).

4. Film dressing according to Claim 3, **characterized in that** the break line (13) in the portion that passes through a grip tab (8), which extends beyond the outline of the plastic film (2), consists of a discontinuous break line.

5. Film dressing according to any of Claims 1 to 4, **characterized in that** the grip tab (7, 8) protrudes from two opposite edges of the dressing.

6. Film dressing according to any of Claims 2 and 5, **characterized in that** a break line (11, 12; 13; 14, 15) extends from the outer edge of each grip tab (7, 8) to the central opening in the backing layer on at least two opposite sides thereof, these break lines being laterally offset in relation to one another so that an imaginary extension of one of these break lines does not coincide with the other break line.

7. Film dressing according to any of Claims 2 to 6, **characterized in that** a curved or wavy break line (11, 12; 13; 14, 15) extends from the outer edge of each grip tab (7, 8) to the central opening in the backing layer.

## Patentansprüche

1. Folienverband, umfassend eine Kunststofffolie (2), die auf einer Seite mit einem hautfreundlichen Klebstoff (3) beschichtet ist, eine Trägerschicht (5), welche an der Kunststofffolie auf der gegenüberliegenden Seite zur klebstoffbeschichteten Seite abnehmbar befestigt ist, und eine an der anhaftenden Beschichtung abnehmbar befestigte Schutzschicht (4),
**dadurch gekennzeichnet, dass** die Trägerschicht (5) zumindest eine Greiflasche (7, 8) umfasst, die von einer Kante des Verbands über die Außenlinie der Kunststofffolie hinausragt, und dass sich eine Bruchlinie (11, 12) von der Außenkante jeder Greiflasche (7, 8) zu einem Mittelteil (6) der Trägerschicht erstreckt, wodurch die Greiflasche in zwei Teile unterteilt wird.

2. Folienverband nach Anspruch 1, **dadurch gekennzeichnet, dass** die Trägerschicht (5) eine mittige Öffnung (6) aufweist.

3. Folienverband nach Anspruch 2, **dadurch gekennzeichnet, dass** die Schutzschicht (4) eine der Außenlinie der Trägerschicht (5) entsprechende Außenlinie aufweist.

4. Folienverband nach Anspruch 3, **dadurch gekennzeichnet, dass** die Bruchlinie (13) in dem Teil, welcher durch eine Greiflasche (8) hindurchgeht, die über die Außenlinie der Kunststofffolie (2) hinausragt, aus einer diskontinuierlichen Bruchlinie besteht.

5. Folienverband nach irgendeinem der Ansprüche 1 bis 4,
**dadurch gekennzeichnet, dass** die Greiflasche (7, 8) aus zwei gegenüberliegenden Kanten des Verbands hervorsteht.

6. Folienverband nach irgendeinem der Ansprüche 2 und 5, **dadurch gekennzeichnet, dass** sich eine Bruchlinie (11, 12; 13; 14, 15) von der Außenkante jeder Greiflasche (7, 8) zur mittigen Öffnung in der Trägerschicht auf zumindest zwei gegenüberliegenden Seiten davon erstreckt, wobei diese Bruchlinien im Verhältnis zueinander seitlich versetzt sind, so dass eine gedachte Verlängerung einer dieser Bruchlinien nicht mit der anderen Bruchlinie zusammenfällt.

7. Folienverband nach irgendeinem der Ansprüche 2 bis 6,
**dadurch gekennzeichnet, dass** sich eine gekrümmte oder wellige Bruchlinie (11, 12; 13; 14, 15) von der Außenkante jeder Greiflasche (7, 8) zur mittigen Öffnung in der Trägerschicht erstreckt.

## Revendications

1. Pansement en film comprenant un film plastique (2), qui est revêtu sur une face d'un adhésif doux pour la peau (3), une couche de support (5), qui est fixée de manière amovible au film plastique sur la face opposée à la face revêtue d'adhésif, et une couche protectrice (4) fixée de manière amovible au revêtement adhésif, **caractérisé en ce que** la couche de support (5) comprend au moins une languette de saisie (7, 8), qui fait saillie à partir d'un bord du pansement au-delà du contour du film plastique, et qu'une ligne de rupture (11, 12) s'étend à partir du bord extérieur de chaque languette de saisie (7, 8) vers une partie centrale (6) de la couche de support, divisant la languette de saisie en deux parties.

2. Pansement en film selon la revendication 1, **caractérisé en ce que** la couche de support (5) présente une ouverture centrale (6).

3. Pansement en film selon la revendication 2, **caractérisé en ce que** la couche protectrice (4) présente un contour correspondant au contour de la couche de support (5).

4. Pansement en film selon la revendication 3, **caractérisé en ce que** la ligne de rupture (13) dans la portion qui passe à travers une languette de saisie (8), qui s'étend au-delà du contour du film plastique (2), se compose d'une ligne de rupture discontinue.

5. Pansement en film selon l'une quelconque des revendications 1 à 4, **caractérisé en ce que** la languette de saisie (7, 8) fait saillie à partir de deux bords opposés du pansement.

6. Pansement en film selon l'une quelconque des revendications 2 et 5, **caractérisé en ce qu'une** ligne de rupture (11, 12; 13; 14, 15) s'étend à partir du bord extérieur de chaque languette de saisie (7, 8) à l'ouverture centrale dans la couche de support sur au moins deux faces opposées de celle-ci, ces lignes de rupture étant latéralement décalées l'une par rapport à l'autre si bien qu'un prolongement imaginaire de l'une de ces lignes de rupture ne coïncide pas avec l'autre ligne de rupture.

7. Pansement en film selon l'une quelconque des revendications 2 à 6, **caractérisé en ce qu'une** ligne de rupture courbée ou ondulée (11, 12; 13; 14, 15) s'étend à partir du bord extérieur de chaque languette de saisie (7, 8) à l'ouverture centrale dans la couche de support.
